(19) Europäisches Patentamt
European Patent Office
Office européen des brevets

(11) Publication number: **0 414 894 A1**

(12) # EUROPEAN PATENT APPLICATION published in accordance with Art. 158(3) EPC

(21) Application number: **89906490.1**

(22) Date of filing: **25.01.89**

(86) International application number:
**PCT/SU89/00026**

(87) International publication number:
**WO 90/08766 (09.08.90 90/19)**

(51) Int. Cl.5: **C07F 9/09**, C07F 9/11, C07F 9/12, C07F 9/14, C07F 9/24, C07F 9/26, A61K 31/675, A01N 57/30

invt.20 Danko, Oleg, Kiev 252148 SUinvt.21 Melnik, Mark, Kiev 252024 invt.22 Matoshko, Gal. Kiev 252110.

(43) Date of publication of application:
**06.03.91 Bulletin 91/10**

(84) Designated Contracting States:
**CH DE FR GB IT LI NL**

(71) Applicant: **KIEVSKY NAUCHNO-ISSLEDOVATELSKY INSTITUT EPIDEMIOLOGII I INFEKTSIONNYKH BOLEZNEI IMENI L.V.GROMASCHEVSKOGO**
**supusk Stepana Razina, 4**
**Kiev, 252038(SU)**

(72) Inventor: **RUDAVSKY, Vladimir Panteleimonovich**
**ul. Bazhova, 10-42**
**Kiev, 252100(SU)**
Inventor: **GORELOV, Stanislav Anatolievich**
**ul. Tupoleva, 6-62**
**Kiev, 252049(SU)**
Inventor: **SADOVSKAYA, Nina Pavlovna**
**ul. Planerskaya, 2/26-1**
**Kiev, 252061(SU)**
Inventor: **MIKHNO, Ivan Leontievich**
**ul. Krasnoarmeiskaya, 97-21**
**Kiev, 252006(SU)**
Inventor: **DEGTYAREV, Georgy Pavlovich**
**ul. Mayakovskogo, 14-24 Moskovskaya obl.**
**Khimki, 141400(SU)**
Inventor: **STAROSTINA, Alla Ignatievna**
**Litovsky bulvar, 3-524**
**Moscow, 117588(SU)**
Inventor: **SAVCHENKO, Vladimir Ivanovich**
**bulvar Zheleznyaka, 21-97**
**Moscow, 125239(SU)**
Inventor: **TELNOV, Nikolai Fedorovich**
**ul. Vuteticha, 9-62**
**Moscow, 125422(SU)**
Inventor: **SHAPIRO, Natalia Ruvimovna**
**Lvovskaya ploschad, 6-5**
**Kiev, 252053(SU)**
Inventor: **SKRIPKA, Ljudmila Ivanovna**
**Vozdukhoflotsky pr., 7-36**
**Kiev, 252049(SU)**
Inventor: **BOIKO, Leonid Danilovich**

**(décédé)(SU)**
Inventor: **TARAN, Valentina Vasilievna**
**ul. Draizera, 9v-126**
**Kiev, 252217(SU)**
Inventor: **SHUMILO, Tamara Vasilievna**
**ul. Moskovskaya, 17/2-204**
**Kiev, 252010(SU)**
Inventor: **BORZUNOV, Evgeny Ermolaevich**
**pr. Geroev Volgograda, 30-24**
**Kiev, 252216(SU)**
Inventor: **POPOVICH, Galina Grigorievna**
**ul. Vasilkovskaya, 2a-140**
**Kiev, 252040(SU)**
Inventor: **STEPANCHUK, Valentin Andreevich**
**ul. Priozernaya, 12a-110**
**Kiev, 252211(SU)**
Inventor: **KUZMENKO, Viktor Pavlovich**
**ul. Krasnotkatskaya, 24-19**
**Kiev, 252094(SU)**
Inventor: **BULGAKOV, Vsevolod Afanasievich**
**ul. Oktyabrskoi revoljutsii, 16-30**
**Kiev, 252021(SU)**
Inventor: **PONOMAREVA, Viktoria Efimovna**
**ul. Karla Marxa, 12-43**
**Kiev, 252001(SU)**



Xerox Copy Centre

(74) Representative: **Spencer, Graham Easdale et al**
**A.A. Thornton & CO Northumberland House**
**303-306, High Holborn**
**London WC1V 7LE(GB)**

(54) **DERIVATIVES OF TRICHLOROPHOSPHASOHYDROCARBONS AND INSECTICIDAL BACTERICIDAL, FUNGICIDAL, VIRICIDAL AND OVOCIDAL PREPARATIONS BASED ON THEM.**

(57) New substances - derivatives of trichlorophosphasohydrocarbons have the following general fomula (I): para-$NO_2C_6H_4CHXCH/CH_2X'/N = P\ R\ R^1R^2$, where X and X' = hydroxyl or chlorine, $R = R^1 = R^2 = OCH_3$ $OC_2H_5$, $OC_3H_7$-H, $OC_3H_7$-izo, $OC_4H_9$-H, $OCH/C_6H_5/CH_2Cl$, formula (II), $OC_6H_4CH_3$-para, $OC_6H_4Cl$-para; where, when $R = 0C_6H_5$, $OC_6H_4Cl$-para, $R^1 = R^2 = Cl$ or $OC_6H_5$; and when $R = NHC_6H_5$, $R^1 = R^2 = Cl$ or $NHC_6H_5$; and when $R = R' = OC_6H_5$, $OC_6H_4Cl$-para, $R^2 = Cl$. The claimed compositions have a biocidal activity and are the active substances of insecticidal, bactericidal, fungicidal, viricidal and ovocidal preparations.

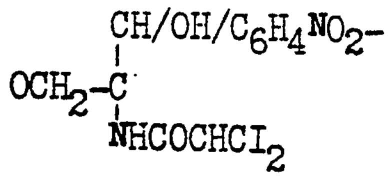
(II)

# TRICHLOROPHOSPHAZOHYDROCARBON DERIVATIVES AND INSECTICIDAL, BACTERICIDAL, FUNGICIDAL, VIRULISIDAL AND OVOCIDAL COMPOSITIONS BASED THEREON

## Field of the Invention

The present invention relates to the field of organic chemistry, more specifically it relates to novel compounds, namely thrichlorophospazohydrocarbon derivatives and insecticidal, bactericidal, fungicidal, virulisidal and ovocidal compositions based thereon.

## Prior Art

Various compounds are known to have an ovocidal activity which are useful as active ingredients for insecticidal, bactericidal, fungicidal, virulisidal and ovocidal compositions. Thus for example 0,0-dimethyl/1-hydroxy-2,2,2,-trichloroethyl/-phosphonate is known to be an active principle of an insecticidal composition / chlorophos, divone, diloxe, trichlorophen/. The composition is used as a 0,1-0,3% aqueous solution and an aerosol/ "Spravochnik po pestitsydam", 1985 g., Moskva, Khimia.

This preparation is unstable, with it being quickly decomposed by light and in an alkaline medium by action of reducing agents, therefore for ots effect to be attained it is necessary that the preparation be applied again in 3-5 days. Using the preparation is unsafety for a human being because of its toxicity ($LD_{50}$ for mice and rats is of 225-1200 mg/kg) and an irritant action. The repeated application of the preparation takes a lot of labour and time, and is not always effective since insects acquire the resistivity to the preparation, Moreover, after applying it an unpleasant, pungent suffocating odour keeps for a long time.

It is known in the art to use a fungicidal composition containing as an active component 2,3-dichloro-1,4-na-phthoquinone /phygon/ to control against phytopathogenic fungi of agricultural crops such as apples, peaches, cherry-trees, beans, tomatoes, rice /"Spravochnik po pestitsidam" 1985, Moskva, Khimia/. This compound is substantially insoluble in acetone, benzene. It is mainly produced in the form of a 50% or 4% powdery composition containing a filling agent for dressing seeds. The composition exerts an irritant action on mucouse coats and skin and is toxic.

It is known in the art to use a fungicidal composition containing zinc N,N-ethylene-bis-dithiocarbamate /zineb/ to control against phytopathogenic fungi of agricultural crops as apples, pears, plums and the life as well as potatoes, tomatoes, cucumbers /"Spravochnik po pestitsidam", 1985, Moskva, Khimia/. The composition is applied by spraying it onto fruit and vegetable crops affected with phytopathogenic fungi in the amount of 1.9 to 6.4 kg/ha. This composition is toxic and irritates skin and mucouse coats. The composition is produced by using a complicated manufacturing processing that makes it economically inefficient.

It is well known in the art a fungicidal composition containing thiuram as an active ingredient to control against phytopathogenic fungi /"Spravochnik po pestitsidam", 1985, Moskva, Khimia/. The composition is used as a seed dresser from fungi or disease exciters of cereals, grain legumes, industrial and vegetable crops, drug and flower plants in the consumption rates of 1.2 to 6.4 kg/ha. The composition is toxic and irritates cutaneous and mucouse coats. A residual content of the composition is unpermissible in food products and a forage fodder.

It is well known in the art a bactericidal and virucidal composition containing as an active ingredient sodium N-chlorohensene sulphonamide. The composition is used for disinfecting rooms, implements, dishware, hands, medicinal instruments, clothes, any garbage, a sanitary equipment, human excrements and the like. The use of the composition, however, is not always efficient because in aqueous solutions the title compound has unstability, a pungent suffocating chlorine odour and irritates the skin as well as the mucosas of eyes and respiratory organs that are accompanied by itching and peeling the skin, an allergic rhinitis, lacrimation, eye discomforts, progressive headaches. All these appearances limits the application of the composition, especially in pulmonological and other medicinal institutions. In carrying out the treatment with the composition it is necessary that respiratory organs and a cutaneous covering of hands be protected as well as special conditions for its storage be set up because under light it is decomposed to release a free chlorine, with its activity decreasing. The composition has a pronounced corrosivity.

An ovocidal carbathione composition is known to contain sodium N-methylthiocarbamate dihydrate as an active ingredient /Khimichesky entsyklopedichesky slovar", 1983, Moskva, "Sovetskaya entsyklopedia", p.242/. The composition prepared in the form of a 40% aqueous solution is used as a soil sterilizer to control against soil pests/ nematodes, microorganisms, insects/ of vegetable and ornamental plants. In the

form of a 2% aqueous solution it is used as a soil desinvasion agent. The composition is employed in a combination with a stabilizing agent, namely a 1% terniary amine. The composition has an unpleasant, strong smell, and irritates the mucosal coats of upper respiratory passages.

Detailed Description of the Invention

Trichlorophosphazohydrocarbon derivatives according to the invention, and insecticidal, bactericidal, fungicidal, virucidal and ovocidal compositions based thereon are novel, and they have not been reported.

The invention is based on the problem of providing novel compounds and compositions based thereon, having high insecticidal, bactericidal, virulicidal, fungicidal, and ovocidal activity, low toxicity, enough stability under storage and application, no side-effects, an excellent solubility in water and other solvents, a wide range of action, produced by a simple production process.

The problem is solved by that novel trichlorophosphazohydrocarbon derivatives according to the invention have the following general formula:

para-$NO_2C_6H_4CHXCH(CH_2X')N$ is $PRR^1R^2$, wherein X and X' are hydroxyl group or Cl, $R = R^1 = R^2$ are $OCH_3$, $OC_2H_5$, $OC_3H_7$-$HOC_3H_7$-iso, $OC_4H_9$-H, $OCH(C_6H_5)CH_2Cl$,

$$OCH_2-CH(NHCOCHCl_2)-CH(OH)C_6H_4NO_2\text{-para}$$

$OC_6H_4CH_3$-para, $OC_6H_4Cl$-para, wherein provided that R is $OC_6H_5$, $OC_6H_4Cl$-para, $R^1 = R^2$ are Cl or $OC_6H_5$; and when R is $NHC_6H_5$, $R^1 = R^2$ are Cl or $NHC_6H_5$; and when $R = R^1$ bare $OC_6H_5$, $OC_6H_4Cl$-para, R is Cl.

The novel compounds of the invention, while being the active ingredients of insecticidal, bactericidal, fungicidal,virulicidal and ovocidal compositions, exhibits a high biocidal activity, a stable effect, a low toxicity, a wide range of action, as well as they have no unpleasant odour and irritating action on a skin coat and the mucosas of eyes and breathing passages.

An insecticidal composition of the invention comprising an active ingredient and a solvent contains as an active ingredient trichlorophosphazohydrocarbon derivatives of the general formula:

para-$NO_2C_6H_4CHXCH/CH_2X'/N = PRR^1R^2$, wherein X and X' is hydroxyl group or Cl, $R = R^1 = R^2$ are $OCH_3$, $OC_2H_5$, $OC_3H_7$-$HOC_3H_7$-iso, $OC_4H_9$-H, $OCH(C_6H_5)CH_2Cl$,

$$OCH_2-CH(NHCOCHCl_2)-CH(OH)C_6H_4NO_2\text{-para}$$

An insecticidal composition according to the invention is characterized by high activity, a stable effect, a low toxicity, having no unpleasant odour and irritating action on the mucosas of eyes or breathing passages and a skin coat. In the composition of the invention an active ingredient is prefered to be present in the amount of 2% by mass, and it is preferable that a solvent be used as a 5% aqueous solution of glucose.

A bactericidal composition of the invention comprising an active ingredient and a solvent, contains as an active ingredient trichlorophosphazohydrocarbon derivatives of the general formula:

para-$NO_2C_6H_4CHXCH/CH_2X'/N = PRR^1R^2$, wherein

X and X' are hydroxyl or chlorine,
$R = R^1 = R^2$ each represent $OCH_3$, $OC_6H_4CH_3$-para, $OC_6H_4Cl$-para, wherein when R is $OC_6H_5$, $OC_6H_4Cl$-para, $R^1 = R^2$ each independently represent chlorine or $OC_6H_5$: and when $R = R^1$ each independently represent $OCH_6H_5$ or $OC_6H_4Cl$, $R^2$ is Cl.

The bactericidal composition according to the invention contains an active ingredient preferably in the amount of 0.5% by mass, and water as a solvent. The bactericidal composition of the invention is characterized by high activity, a stable action to Gram-positive and Gram-negative vegetative microorganisms, a low toxicity to mammalians, having no pungent, unpleasant odour, its effective concentrations cause no irritating effect on a skin coat, the mucosas of eye and breathing passages. Moreover, the composition exerts no sensitizing effects on a human organism.

A fungicidal composition of the invention comprising an active ingredient and a solvent contains as an active ingredient trichlorophosphazohydrocarbon derivatives of the general formula:
para-$NO_2C_6H_4CHXCH/CH_2X'/N = PRR^1R^2$, wherein
X and X' are hydroxyl or chlorine, $R = R^1 = R^2$ each independently represent $OCH_3$, $OC_2H_5$.

In the fungicidal composition of the invention preferably contains an active ingredient in the amount of 0.01% by weight, and water as a solvent.

The fungicidal composition of the invention is characterized by a high activity, stability of action to Candia albicans, Candia tropicalis, a low toxicity to warm-blooded animals, having no pangent unpleasant odour, its effective concentrations exert no irritating action on skin coats, the mucosas of eyes and breathing passages.

The virulioidal composition of the invention comprising an active ingredient and a solvent, wherein an active ingredient is trichlorophosphazohyrdocarbon derivatives of the general formula:

para-$NO_2C_6H_4CHXCH/CH_2X'/N = PRR^1R^2$, wherein
X and X' are hyrdoxyl or chlorine, $R = R^1 = R^2$ each independently represent $OC_2H_5$, $OC_3N_7$-H, $OC_3H_7$-iso, $OC_4H_9$-H, $OCH/C_6H_5/CH_2Cl$,

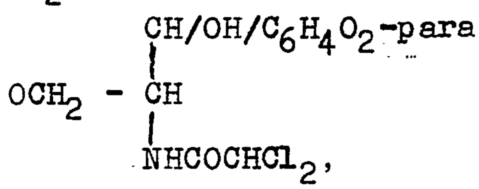

when R is $OC_6H_5$, $OC_6H_4Cl$-para, $R^1 = R^2$ each independently represent Cl or $OC_6H_5$; and when $R = R^1$ each independently represent $OC_6H_5$, $OC_6H_4Cl$-para, $R^2$ is Cl.
The virulicidal composition according to the invention contains an active ingredient in the amount of 0.1 to 2.0% by mass, and as a solvent it contains a 0.85% NaCl solution.

The virulicidal composition of the invention exibits a high virulicidal activity to hepatitis B virus, influenza viruses and meningopneumonia pathogene as well as it has no irritating action on skin and mucouse coats, and no unpleasant odour.

The ovocidal composition according to the invention comprising an active ingredient and a solvent characterized in that as an active ingredient it contains trichlorophosphazohydrocarbon derivatives of the general formula:

para-$NO_2C_6H_4$ $CHXCH/CH_2X'/N = PRR^1R^2$, wherein
X and X' are hydroxyl or chlorine, $R = R^1 + R^2$ each independently represent $OC_6H_5$,

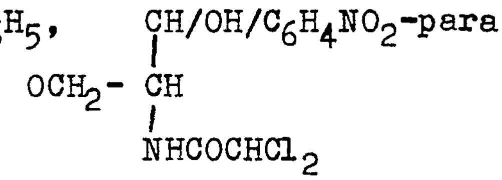

and when R is $NHC_6H_5$, $R^1 = R^2$ each independently represent Cl or $NHC_6H_5$.

The ovocidal composition of the invention contains an active ingredient preferably in the amount of 2% by mass, and as a solvent it preferably contains water.

The ovocidal composition of the invention has a high ovocidal activity which is superior to a known carbathione.

Description of Prefered Embodiment

The novel trichlorophosphazohyrdocarbon derivatives are crystalline silids or yellow viscous liquids, soluble in water, alcohols, acetone, benzene, toluene, and other organic solvents.

In taking IR-spectra of the compounds according to the invention characteristic absorption lines for N = P are in the range of 1330-1360 $cm^{-1}$, $^{31}p$ NMR spectrum is of 1.76-1.77 ppm.

The compounds of the invention have an insecticidal, bactericidal, virulicidal, fungicidal and ovocidal activity. These properties of the compounds according to the invention are determined in biological tests by a direct contact of solutions of the claimed compositions with synanthropic insects, viruses, bacteria, helminthic eggs and fungi.

Compositions of the invention cause the death of room flies, and other synanthropic insects such as entheroviruses, hepatitis B viruses, influenza viruses and the like.

All the compounds and the compositions based thereon which are now claimed by us have a high biocidal activity as well as they are characterized by a stable effect, a low toxicity, having no unpleasant odour and irritating action on the mucosas of eyes and airways. Moreover, they have a wide range of action on a variety of biological objects. The compounds of the invention are prepared by a simple production processing. The claimed novel compounds trichlorophosphazohydrocarbon derivatives are phosphazohydrocarbon esters and amides. They are prepared by reacting trichlorophosphazohydrocarbons with alcohols in the ratio of 1:3 and with phenols in the ratios of 1:1. 1:2, 1:3 in the presence of bases. For trichlorophosphazohydrocarbon amides to be prepared trichlorophosphazohydrocarbons are reacted with amines in the ratio of 1:2 or 1:6 in a benzene solution. The resultant reaction mixture is allowed to stay at room temperature for a long time. Thereafter, amine or triethylamine hydrochloride and benzene are removed from the solution. The end product is obtained as a crystalline solid or yellow viscous liquid having a fruit-vinous odour, well-soluble in water, alcohols, acetone, benzene, toluene, and other organic solvents.

For a better undestanding of the present invention the following examples of producing the novel compounds, compositions based thereon and tests for their activities are given herein below.

Example 1

To a solution containing 0.03 mol of methyl alcohol and 0.03 mol triethylamine in 30 ml of benzene a solution of trichlorophosphazo-1-para-nitrophenyl-1,3-propandiol in 20 ml of benzene was gradually added thereto under a continuous stirring. The reaction mixture was allowed to stay for 24 hours at 20°C. The formed triethylamine hydrochloride was sucked off. Benzene was evaporated in vacuum. The residue containing the product as a viscous substance which was purified by reprecipitation with a petroleum ether from a benzene solution in the presence of an activated charcoal. As a result, there was obtained the end trimethoxyphosphazo-1-para-nitrophenyl-1,3-propandiol of the formula:
para-$NO_2C_6H_4CHOHCH/CH_2OH/N = P/OCH_3/_3$, which was a viscous liquid, soluble in water, alcohols, acetone. The yield of the end product was 84% by mass.

Elemental analysis for $C_{12}H_9N_2O_7P$.
Found, %: C 43.04, H 5.43, N 8.80, P 9.12.
Calculated, %: C 43.10, H 5.72, N 8.40, P 9.28.
IR spectrum for N = P: 1340 $cm^{-1}$.

The obtained compound had an insecticidal activity. The compound obtained was used for preparing an insecticidal composition which was a 2% solution of the compound in a 5% aqueous solution of glucose. The test for activity of the insecticidal composition of the invention was conducted on domestic flies. Into gause 10x10 cm boxes /6 boxes/ 6-7-day flies were placed in the number of 50 individuals per box, which were of both sexes. The Petri dishes containing the insecticidal composition of the invention were put into 5

boxes. The observations for fly death were carried out in 48-72 hours. The Petri dish containing a 5% aqueous glucose solution were placed into a control box. The test for an insecticidal activity of the composition according to the inventicn was carried out in comparison with a conventional insecticidal composition based on 0,0-dimethyl/1-hydroxy-2,2,2-trichloroethyl/phosphonate.

The full-scale tests for activity of the insecticidal composition of the invention to domestic flies in habitable and industrial premises were carried out as follows. The Petri dishes containing a 2% solution of the insecticidal composition of the invention were located according 2-3-dishes per 20 $m^2$. The composition activity was evaluated by a direct estimation of killed insects in 72 hours or by using an adhesive tape to count the number of flies on its surface prior to and after conducting the test. The test data are given in Table 1.

Example 2

The process was carried out in a manner similar to that of Example 1 except that 0.03 mol of ethyl alcohol was used instead of methyl alcohol. As a result, there was obtained triethoxyphosphazo-1-para-nitrophenyl-1,3-propandiol of the formula:
para-$NO_2C_6H_4CHOHCH/CH_2OH/N$ = $P/OC_2H_5/_3$, which was a viscous liquid, soluble in water, alcohols, acetone.

The yield of the end product was 83% by mass.

Elemental analysis for $C_{15}H_{25}N_2O_7P$.
Found, %: C 47.43, H 7.35, N 7.43, P 8.30.
Calculated, %: C 47.40, H 7.38, N 7.35, P 8.15.

IR spectrum for N = P: 1335 $cm^{-1}$.

The compound had an insecticidal activity. It was used for preparing the insecticidal composition which was a 2% solution containing the compound obtained in a 5% aqueous glucose solution.

The test for the composition activity was carried out in a manner similar to that of Example 1. The test data are given in Table 1.

Example 3

The process was carried out in a manner similar to that of Example 1 except that 0.03 mol of isopropyl alcohol was used instead of methyl alcohol. As a result, there was obtained the end product namely triisopropoxyphosphazo-1-para-nitrophenyl-1,3-propandiol of the formula:

para-$NO_2C_6H_4CHOHCH/CH_2OH/N$ = $P/OC_3H_7$-iso/$_3$, which was a viscous liquid, soluble in water, alcohols, acetone.

The yield of the end product was 91% by mass.

Elemental analysis for $C_{18}H_{31}N_2O_7P$.
Found, %: C 51,52, H 7.35, N 6.30, P 7.20.
Calculated, %: C 51.68, H 7.47, N 6.70, P 7.38.
IR spectrum for N = P: 1330 $cm^{-1}$.

The compound had an insecticidal activity. The test of the composition based on the compound was carried out in a similar manner as in Example 1. The test data are given in Table 1.

Example 4

The process was carried out similarly to that of Example 1, except that 0.03 mol of 2-chloro-1-phenylethyl alcohol was used instead of methyl alcohol. As a result, there was obtained the end product namely tris/2-chloro-1-phenylethoxy/phosphazo-1-para-nitrophenyl-1,3-propandiol of the formula:

para-$NO_2C_6H_4CHOHCH/CH_2OH/N=P[OCH(C_6H_5)CH_2Cl]_3$, which is a viscous liquid, soluble in water, alcohols, and acetone. The yield of the end product was 88% by mass.

Elemental analysis for $C_{33}H_{34}Cl_3N_2O_7P$.

Found, %: C 56.87, H 4.77, Cl 14.47, N 4.20, P 4.37.
Calculated, %: C 56.76, H 4.76, Cl 14.77, N 3.90, P 4.30.
IR spectrum for N = P: 1340 $cm^{-1}$.

IR spectrum for N = P: 1340 $cm^{-1}$.
The compound had an insecticidal activity. The test of the composition based thereon was carried out according to a manner similar to that of Example 1. The test data are given in Table 1.

Example 5

The process was carried out in a manner similar to that of Example 1. 0.03 mol of propyl alcohol and 0.01 mol of trichlorophosphazo-1-para-nitrophenyl-1-chloro-3-propanol were used as the starting compounds, the ramaining reagents were the same as in Example 1. The obtained product namely tri-n-propoxyphosphazo-1-para-nitrophenyl-1-chloro-3-propanol of the formula:

para-$NO_2C_6H_4CHOHCH/CH_2Cl/N=P/OC_3H_7$-H/$_3$ was a yellow liquid, which was soluble in water, acetone, benzene, toluene. The yield of the end product was 91% by mass.

Elemental analysis for $C_{18}H_{30}ClN_2O_6P$.
Found, %: C 49.31, H 7.01, Cl 8.01, N 6.14, P 7.10.
Calculated, %: C 49.49, H 6.93, Cl 8.11, N 6.41, P 7.09.
IR spectrum for N=P: 1350 $cm^{-1}$.

The compound had an insecticidal activity. The te st of the composition based thereon was carried out in a manner similar manner to that of Example 1. The test results are given in Table 1.

Example 6

The process was carried out in a manner similar to that described in Example 1, except that 0.03 mol of isopropyl alcohol and 0.01 mol of trichlorophosphazo-1-paranitrophenyl-1-chloro-3-propanol were taken as the starting compounds. As a result, there was obtained the end product or tri-isopropoxyphosphazo-1-para-nitrophenyl-1-chloro-3-propanol of the formula:

para-$NO_2C_6H_4CHOHCH/CH_2Cl/N=P/OC_3H_7$-iso/$_3$, which was a yellow crystalline substance, soluble in water, alcohols, acetone, benzene, toluene. The yield of the end product was 89% by mass, m.p. 137-138 °C.

Elemental analysis for $C_{18}H_{30}ClN_2O_6P$
Found. %: C 49.28, H 6.83, Cl 8.19, N 6.28, P 6.98.
Calculated, %: C 49.49, H 6.93, Cl 8.11, N 6.41. P 7.09..
IR spectrum for N = P: 1355 $cm^{-1}$.

The compound had an insecticidal activity. The test of the composition based thereon was carried out

in a manner similar to that described in Example 1. The test results are given in Table 1.

Example 7

The process was carried out in a manner similar to that of Example 1 except that 0.03 mol of butyl alcohol was used instead of methyl alcohol. As a result, there was obtained the end product namely tri-n-butoxyphosphazo-1-paranitrophenyl-1-chloro-3-propanol of the formula:

para-$NO_2C_6H_4CHOHCH/CH_2Cl/N=P/OC_4H_9$-H/3, which was a viscous liquid, soluble in water, alcohols, acetone. The yield of the end product was 94% by mass.

Elemental analysis for $C_{21}H_{36}ClN_2O_6P$
Found, %: C 52.60, H 7.48, Cl 7.32, N 5.96, P 6.50.
Calculated, %: C 52.55, H 7.57, Cl 7.40, N 5.85, P 6.46.
IR spectrum for N=P: 1355 $cm^{-1}$.

The compound had an insecticidal activity. The test of the composition based thereon was carried out similarly to that of Example 1. The test data are given in Table 1.

Example 8

The process was carried out in a manner similar to that described in Example 1 except that 0.03 mol of 2-chloro-1-phenyl ethyl alcohol was used instead of methyl alcohol. As a result, there was obtained the end product, namely tris/2-chlorol-phenylethoxy/-phosphazo-1-paranitrophenyl-1-chloro-3-propanol of the formula:

para-$NO_2C_6H_4CHOHCH/CH_2Cl/N=P[OCH(C_6H_5)CH_2Cl]_3$, which was a viscous liquid, soluble in water, alcohols, acetone. The yield of the end product was 96% by mass.

Elemental analysis for $C_{33}H_{33}Cl_4N_2O_6P$
Found, %: C 64.02, H 5.29, Cl 5.68, N 4.48, P 5.01.
Calculated, %: C 63.92, H 5.36, Cl 5.72, N 4.52, P 5.00.
IR spectrum for N=P: 1360 $cm^{-1}$.

The compound had an insecticidal activity. The test of the composition based thereon was carried out in a manner similar to that described in Example 1. The test results are given in Table 1.

Example 9

The process was carried out in a manner similar to that of Example 1 except that 0.03 mol of methyl alcohol and 0.01 mol of trichlorophosphazo-1-para-nitrophenyl-1,3-dichloropropane were used as the starting compounds. As a result, there was obtained the end product or trimethoxyphosphazo-1-para-nitrop henyl-1,3-dichloropropane of the formula:

para-$NO_2C_6H_4CHOHCH/CH_2Cl/N=P/OCH_3$/3, which was a yellow crystalline substance, soluble in water, alcohols, acetone, benzene, toluene. The yield of the end product was 84% by mass, m.p. 94-95° C.

Elemental analysis for $C_{12}H_{17}Cl_2N_2O_5P$
Found, %: C 38.77, H 4.51, Cl 19.05, N 7.23, P 8.32.
Calculated, %: C 38.81, H 4.58, Cl 19.13, N 7.55, P 8.36.
IR-spectrum for N=P: 1350 $cm^{-1}$.

The compound had an insecticidal activity. The test of the composition based thereon to evaluate an

insecticidal activity was carried out in a manner similar to that described in Example 1. The obtained results are given in Table 1.

This compound had also a bactericidal activity. Based on the obtained compound, there was prepared a bactericidal composition which was a 0.5% aqueous solution containing it. Tests for bactericidal activity of the bactericidal composition according to the invention were conducted by desinfection of lawn-made test objects as well as the suspended culture method to standard strains from Escherichia coli and Staphylococcus aureus. The tests were carried out under protein-free and protein loadings. The lawn test objects that were seeded with a suspensions culture containing $2x10^9$ cells of E, coli or S. aureus, were placed into the Petri dishes based on 2-dishes per each exposition at the composition rate of 0.5 ml per 1 test object. On the expiry of exposition time of the composition on microorganisms, test objects were washed twice in a sterile water and transfered into a beef-extract broth. The seedings were placed into a thermostat at 37° C. The results were recorded in 24.48 hours and 7 days to differentiate bacteriostatic and bactericidal activities of the composition. A similar test was conducted for activity of the composition containing sodium N-chlorobenzene sulfamide. The activity of the composition was estimated by counting vital test microorganisms after each exposition of a 0.5% concentration of the composition. The test results are given in Table 2.

Example 10

The process was carried out in a manner similar to that described in Example 1 except that 0.03 mol of ethyl alcohol was used instead of methyl alcohol. As a result, there was obtained the end product namely triethoxyphosphazo-1-para-nitrophenyl-1,3-dichloropropane of the formula:

para-$NO_2C_6H_4CHClCH/CH_2Cl/N$ = $P/OC_2H_5/_3$, which was a crystalline substance, soluble in water, alcohols, acetone, benzene, toluene. The yield of the end product was 94% by mass, m.p. 105-107° C.

Elemental analysis for $C_{15}H_{23}Cl_2N_2O_5P$
Found, %: C 43.33, H 5.51, Cl 17.15, N 6.72, P 7.52.
Calculated, %: C 43.58, H 5.58, Cl 17.19, N 6.79, P 7.52.
IR spectrum for N = P: 1360 $cm^{-1}$, $^{31}P$ NMR spectrum 1,77 ppm.

The compound had an insecticidal and bactericidsl activity. The test of the composition based thereon was carried out in a manner similar to that described in Example 1. The test data are given in Table 1. The test of the composition for a bactericidal activity was carried out according to that of Example 9. The test results are given in Table 2.

Example 11

The process was carried out in a manner similar to that described in Example 1 except that 0.03 mol of propyl alcohol was used instead of methyl alcohol. The obtained end product or tri-n-propoxyphosphazo-1-para-nitrophenyl-1,3-dichloropropane of the formula:

para-$NO_2C_6H_4CHClCH/CH_2Cl/N$ = $P/OC_3H_7$-$H/_3$

was a yellow crystalline substance, soluble in water, alcohols, acetone, benzene, toluene. The end product yield was 90% by mass, m.p. 173-174° C.

Elemental analysis for $C_{18}H_{29}Cl_2N_2O_5P$
Found, %: C 47.31, H 6.30, Cl 15,47, N 6.10, P 6.75.
Calculated, %: C 47.48, H 6.42, Cl 15.55, N 6.14, P 6.79.
IR spectrum for N=P: 1345 $cm^{-1}$.

The compound had an insecticidal activity. The test of the composition based thereon was carried out similarly to that described in Example 1. The test data are given in Table 1.

Example 12

The process was carried out in a manner similar to that described in Example 1 except that 0.03 mol of isopropyl alcohol was used instead of methyl alcohol. The obtained end product namely triisopropoxyphosphazo-1-paranitrophenyl of the formula:

para-$NO_2C_6H_4CHClCH/CH_2Cl/N=P/OC_3H_7$-iso/ was a yellow crystalline substance, soluble in water, alcohols, acetone toluene. The yield of the end product was 88% by mass, m.p. 157-158° C.

Elemental analysis for $C_{18}H_{29}Cl_2N_2O_5P$
Found, %: C 47.39, H 6.38, Cl 15.52, N 6.13, P 6.80.
Calculated, %: C 47.48, H 6.42, Cl 15.55, N 6.14, P 6.79.
IR spectrum for N=P : 1355 $cm^{-1}$.

The compound had an insecticidal activity. The test of the composition based thereon was carried out in a manner similar to that of Example 1. The test results are given in Table 1.

Example 13

The process was carried out in a manner similar to that described in Example 1 except that 0.03 mol of butyl alcohol was used instead of methyl alcohol. The obtained end product manely tri-n-butoxyphosphazo-1-para-nitrophenyl-1,3-dichloropropane of the formula:

para-$NO_2C_6H_4CHClCH/CH_2CL/N=P/OC_4H_9$-H/3 was a yellow crystalline substance, soluble in water, alcohols, acetone, benzene, toluene. The yield of the end product was 89% by mass, m.p. 135-136° C.

Found, %: C 50.53, H 7.12, Cl 14.13, N 5.54, P 6.22.
Calculated, %: C 50.71, H 7.09, Cl 14.25, N 5.62, P 6.22.
IR spectrum for N=P: 1350 $cm^{-1}$.

The compound had an insecticidal activity. The test composition based thereon was carried out similarly to that described in Example 1. The results obtained are diven in Table 1.

Example 14

The process was carried out in a manner similar to that described in Example 1 except that 0.03 mol of 2-chloro-1-phenyl ethyl alcohol was used instead of methyl-alcohol. The obtained end product or tris-/2-chloro-1-phenoxy/phosphazo-1-para-nitrophenyl-1,3-dichloropropane of the formula:

para-$NO_2C_6H_4CHClCH/CH_2Cl/N=P[OCH(C_6H_5)CH_2Cl]_3$ was a yellow substance, soluble in water,alcohols, acetone, benzene, toluene. The yield of the end product was 92% by mass, m.p. 115-116° C.

Found, %: C 53.92, H 4.23, Cl 23.62, N 3.72, P 4.12.
Calculated, %: C 53.95, H 4.29, Cl 23.42, N 3.70, P 4.09.
IR spectrum for N=P : 1360 $cm^{-1}$.

The compound had an insecticidal activity. The test of the composition based thereon was carried out in a manner similar to that described in Example 1. The test data are given in Table 1.

Example 15

The process was carried out in a manner similar to that described in Example 1 except that 0.03 mol of

methyl alcohol and 0.01 mol of trichlorophosphazo-1-paranitrophenyl-1-chloro-3-propanol were used as the starting compounds. As a result, there was obtained the end product manely trimethoxy-1-para-nitrophenyl-1-chloro-3-propanol of the formula:

para-$NO_2C_6H_4CHOHCH/CH_2Cl/N=P/OCH_3/_3$, which was a yellow crystalline substance, soluble in water, alcohols, acetone, benzene, toluene. The yield of the end product was 90% by mass, m.p. 76-78° C.

Elemental analysis for $C_{12}H_{18}Cl\,N_2O_6P$
Found, %: C 40.24, H 5.21, Cl 9.97, N 7.96, P 8.98.
Calculated, %: C 40.86, H 5.14, Cl 10.05, N 7.95, P 8.78.
IR spectrum for N=P: 1355 $cm^{-1}$.

The compound had a fungicidal activity.

A fungicidal composition based on this compound was prepared, which was a 0.01% solution of the compound in water. The fungicidal activity of the composition according to the invention was tested for inhibiting the growth of fungi in Laboratory conditions such as Candia albicans, Candia tropicalis which are a causative factor for candidiasis affecting mucous and skin coats, and internals. For testing it the Petri dishes containing Sabourand growth medium were inoculated by using suspension of microorganisms containing $5x10^7$ cells of fungi in 1 ml.

The restricted surface areas (7 mm in diameter) were coated with the composition by means of a small metal spatula 4x4 mm in size. In a day of incubating it at 37° C in the thermostat the composition inhibited the growth of yeast-like fungi. An antifungal activity of the composition was specified according to the method of 10-fold serial dilution using Sabourand's liquid medium in 2 ml of which $2x10^5$ cells were introduced.

Table 1

Insecticidal activity of the composition according to the invention compared to a known composition and the control

| Nos | Compositions | Death of flies, % Tests in laboratory | large-scale |
|---|---|---|---|
| 1. | The insecticidal composition of the invention according to Example 1 | 89.1 | 87.0 |
| 2. | The insecticidal composition of the invention according to Example 2 | 84.7 | 83.0 |
| 3. | The insecticidal composition of the invention according to Example 3 | 82.0 | 80.0 |
| 4. | The insecticidal composition of the invention according to Example 4 | 73.4 | 75.0 |
| 5. | The insecticidal composition of the invention according to Example 5 | 94.5 | 93.0 |
| 6. | The insecticidal composition of the invention according to Example 6 | 90.9 | 89.0 |
| 7. | The insecticidal composition of the invention according to Example 7 | 96.0 | 96.8 |
| 8. | The insecticidal composition of the invention according to Example 8 | 84.3 | 84.0 |

Continued

| 1 | 2 | 3 | 4 |
|---|---|---|---|
| 9. | The insecticidal composition of the invention according to Example 9 | 85.2 | 83.0 |
| 10. | The insecticidal composition of the invention according to Example 10 | 89.9 | 88.0 |
| 11. | The insecticidal composition of the invention according to Example 11 | 89.0 | 89.0 |
| 12. | The insecticidal composition of the invention according to Example 12 | 92.0 | 90.0 |
| 13. | The insecticidal composition of the invention according to Example 13 | 92.0 | 91.0 |
| 14. | The insecticidal composition of the invention according to Example 14 | 71.3 | 70.0 |
| 15. | The known insecticidal composition based on 0.0-dimethyl-/1-hydroxy-2,2,2-trichloroethyl/ phosphonate | 90.0 | 88.0 |
| 16. | Control | 0.0 | 0.0 |

Table 2

Bactericidal activity of the composition according to the invention compared with a known composition and the control

| Nos | Compositions | Death of micro-organisms, % | |
|---|---|---|---|
| | | Escherichia coli | Staphylococus aureus |
| 1. | The bactericidal composition of the invention according to Example 9 | 100.0 | 99.99 |
| 2. | The bactericidal composition of the invention according to Example 10 | 99.99 | 99.83 |
| 3. | Known composition containing sodium N-chloro-benzene sulfamide | 100.0 | 99.99 |
| 4. | Control | 0.0 | 0.0 |

After incubating for 2 days at 34°C in the thermostat a minimum dilution of the compound exhibiting a fungicidal action was determined.

The test results are given in Table 3.

The large-scale tests were carried out by using Candida albicans and Candida tropicalis strains, with their 2-day agar culture being diluted with an isotonic sodium chloride solution up to the concentration of $5x10^7$ cells in 1 ml. The culture suspension was laid on the surface (7 mm in diameter) of articles made of ceramics, metal, wood and rubber as well as a cotton, flannel and woollen cloth which were presterilized.

In a day after incubation from the treated areas washings were taken with a cotton-gauze tampon which was moistened with Sabouraud's liquid medium, then they were immersed into the tubes containing a similar medium, and placed into thermostat at 37°C for 2 days. Thereafter, the inoculum was transfered onto a solified Sabouraud agar, and the values of growth were recorded according to a conventional procedure. The test data of the composition according to the invention over a known composition (zineb) and the control are given in Table 3.

Example 16

The process was carried out in a manner similar to that descibed in Example 1 except that ethyl alcohol and trichlorophosphazo-1-para-nitrophenyl-1-chloro-3-propanol were used as the starting compounds. The obtained end product namely triethoxy-phosphazo-1-para-nitrophenyl-1-chloro-3-propanol of the formula:

para-$NO_2C_6H_4CHOHCH/CH_2Cl/N=P/OC_2H_5/_3$ was a yellow crystalline substance, soluble in water, alcohols, acetone, benzene, toluene. The yield of the end product was 95% by mass, m.p. 101-103°C.

Elemental analysis for $C_{15}H_{24}Cl.N_2O_6P$
Found, %: C 45.58, H 6.01, Cl 9.05, N 7.16, P 7.81.
Calculated, %: C 45.63, H 6.13, Cl 8.98, N 7.10, P 7.86.
IR spectrum for N=P: 1360, $^{31}P$ NMR spectrum : 1.76 ppm.

The compound had an fungicidal activity. The te st of the composition based thereon was carried out according to a manner similar to that described in Example 15. The test results are given in Table 3.

Example 17

Similarly to that described in Example 1, there was obtained trimethoxyphosphazo-1-para-nitrophenyl-1.3-propandiol. The compound had a fungicidal activity. The test of the composition based thereon was carried out in a manner similar to that of Example 15. The results of tests are given in Table 3.

Example 18

Triethoxyphosphazo-1-para-nitrophenyl-1,3-propandiol was obtained in a manner similar to that described in Example 1. The compound had a fungicidal activity. The test of the composition based thereon was carried out in a manner similar to that of Example 15. The results of these tests are given in Table 3.

Example 19

Similarly to that described in Example 1 there was obtained trimethoxyphosphazo-1-para-nitrophenyl-1,3-dichloropropane. The compound had a fungicidal activity. The test of the composition based thereon was carried out in a manner similar to that described in Example 15. The test results are given in Table 3.

Example 20

According to a manner similar to that described in Example 1 there was obtained triethoxyphosphazo-1-paranitrophenyl-1,3-dichloropropane. The compound had a fungicidal activity. The test of the composition based thereon was conducted according to a manner similar to that described in Example 15. The test data are given in Table 3.

Example 21

The process was carried out in a manner similar to that described in Example 1 except that 0.03 mol of phenol and 0.01 mol of trichlorophosphazo-1-para-nitrophenyl-1,3-propanol were used as the starting compounds. As a result, there was obtained the end product namely triphenoxyphosphazo-1-para-nitrophenyl-1,3-propanol of the formula:

para-$NO_2C_6H_4CHOHCH/CH_2OH/N=P/OC_6H_5/_3$, which was a yellow viscous liquid, being reprecipitated from a benzene solution with petroleum ether in the presence of an activated charcoal, soluble in water, alcohols, acetone, benzene, toluene. The yield of the end product was 85% by mass.

Elemental analysis for $C_{27}H_{25}N_2O_7P$
Found, %: C 61.98, H 4.72. N 5.42, P 5.51.
Calculated, %: C 62.33, H 4.84, N 5.38, P 5.96.
IR spectrum for N = P: 1330 $cm^{-1}$.

The compound obtained had a virucidal activity. Based on the compound there was prepared a virucidal

composition which was a 2% or 1% solution of the compound in a 0.85% sodium chloride solution. A virucidal activity of the composition of the invention was tested against hepatitis B virus, influenza A/$H_1N_1$/ viruses and meningopneumonic pathogenes, causing the most mass-scale attacks of human virus infections. For testing it the virucidal composition of the invention was added into blood serum of a patient or carrier of hepatitis B virus having the starting HBsAg titer of 1:3 to 1:64. After a 30 and 60 minute exposure of it HBsAg titer was determined according to a conventional immunologic method by serial dilutions with a test antigene-antibody system. To the control serum samples 0.85% sodium chloride solution was added at a

table ratio instead of the test composition. A virulicidal activity of the composition according to the invention against hepatitis B viruses was tested over a known. composition containing sodium N-chlorobenzenesulfamide.

A virulicidal activity of the composition according to the invention to influenza viruses was tested in a similar manner by adding 1-2% solution of the composition to a virus-containing allantoic fluid. The virus titer was determined by a conventional method such as hemagglutination--inhibition reaction,

The activity of the composition according to the invention was tested over a known composition containing sodium N-chlorobenzenesulfamide.

The test data are given in Table 4.

Table 3

Fungicidal activity of the composition according to the invention over a known composition and the control

| Nos. | Compositions | Growth inhibition of fungal colonies, % | | | |
|---|---|---|---|---|---|
| | | Candida albicans | | Candida Tropicalis | |
| | | Laboratory tests | Large-scale tests | Laboratory tests | Large-scale tests |
| 1. | The fungicidal composition of the invention according to Example 15 | 100.0 | 100.0 | 100.0 | 100.0 |
| 2. | The fungicidal composition of the invention according to Example 16 | 100.0 | 100.0 | 100.0 | 100.0 |
| 3. | The fungicidal composition of the invention according to Example 17 | 100.0 | 100.0 | 100.0 | 100.0 |
| 4. | The fungicidal composition of the invention according to Example 18 | 100.0 | 100.0 | 100.0 | 100.0 |
| 5. | The fungicidal composition of the invention according to Example 19 | 100.0 | 100.0 | 100.0 | 100.0 |
| 6. | The fungicidal composition of the invention according to Example 20 | 100.0 | 100.0 | 100.0 | 100.0 |
| 7. | A known composition (zineb) | 100,0 | 100,0 | 100.0 | 100,0 |
| 8. | Control | 0.0 | 0.0 | 0,0 | 100.0 |

Example 22

According to a manner similar to that described in Example 1, there was obtained tri-n-propoxyphosphazo-1--para-nitrophenyl-1-chloro-3-propanol. The .compound had a virulicidal activity. A virulicidal activity of the composition containing the compound against influenza A viruses and hepatitis B viruses was tested in a manner similar to that described in Example 21.

A virulisidal activity of the composition according to the invention to meningopneumonia pathogenes

was tested as follows.

To a suspended yolk cell culture of meningopneumonia pathogene/MP strain/ a solution containing the composition of the invention at its concentration of 0.1-0.2% by mass was added. The original infectivity titer was of $10^{-3}$LD 100/0.1 ml for white mice. In 30 and 60 minutes of exposure a neutralizing agent (0.1% sulphanole solution) was added and an infectivity titer for white mice was determined. The animals were observed for 24 days. The test results are given in Table 4.

Example 23

According to a manner similar to that described in Example 1, there was obtained tri-isopropoxyphosphazo-1-para-nitrophenyl-1-chloro-3-propanol. The compound had a virulicidal activity. The composition of the invention based thereon based thereon was tested in a manner similar to that described in Example 22. The experimental data are given in Table 4.

Example 24

According to a manner similar to that described in Example 1 there was obtained tri-n-butoxyphosphazo-1-paranitrophenyl-1,3-dichloropropane. The compound had a virulicidal activity. The composition based thereon was tested similar,to that of Example 22. The test data are given in Table 4.

Example 25

According to a manner similar to that described in Example 1 there was obtained tris/2-chloro-l-phenylethoxy/ phosphazo-1-para-nitrophenyl-1,3-dichloropropane. The compound had a virulicidal activity. The composition based thereon was tested according to a manner similar to that described in Example 22. The experimental data are given in Table 4.

Example 26

According to a manner similar to that described in Example 1, there was obtained triethoxyphosphazo-1-paranitrophenyl-1-chloro-3-propanol. The compound had a virulicidal activity. The composition based thereon was tested in a manner similar to that described in Example 22. The test data are given in Table 4.

Example 27

The process was carried out in a manner similar to that as described in Example 1 except that 0.03 mol of D(-)-threo-1-para-nitrophenyl-2-dichloroacetamido-1,3-propandiol was used instead of methyl alcohol.

The obtained and product namely tris-[D(-)-threo-3-para-nitrophenyl-2-dichloroacetamido-2-hydroxypropoxyphosphazo-L(+)-threo-1-para-nitrophenyl-1,3-dihydroxyiso propyl of the formula:

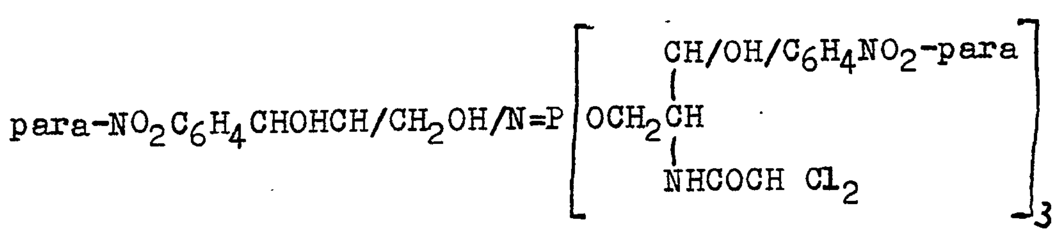

was a crystalline substance, soluble in water, alcohols, acetone. The yield of the end product was 72% by mass, m.p. 164-166°C.

Found, %: C 41.36, H 3.63, Cl 17.58, N 9.05, P 2.41.
Calculated, %: C 41.77, H 3.58, Cl 17.61, N 9.27, P 2.56.
IR spectrum for N=P: 1350 $cm^{-1}$.

The compound had a virulicidal activity. The composition based thereon was tested against influenza A viruses accodring to a manner similar to that described in Example 21. The test data are given in Table 4.

Example 28

The process was carried out in a manner similar to that described in Example 1 except that 0.01 mol of trichlorophosphazo-1-para-nitrophenyl-1-hydroxy-3-chloroisopropyland 0.03 mol of D(-)-threo-1-para-nitrophenyl-2-dichloroacetamido-1,3-propanediol were used as the starting compounds. The obtained end product namely tris-[D(-)-threo-3-para-nitrophenyl-2-dichloroacetamido-2-hydroxypropoxy] phosphazo-L(+)-threo-1-para-nitroplenyl-1-hydroxy 3-chloroisopropyl of the formula:

$$\text{para-}NO_2C_6H_4CHOHCH/CH_2Cl/N{=}P\left[OCH_2\underset{\displaystyle NHCOCHCl_2}{\overset{\displaystyle CH/OH/C_6H_4NO_2\text{-para}}{CH}}\right]_3$$

was a crystalline substance, soluble in water, alcohols, - acetone. The yield of the end product was 85% by mass, m.p. 182-184° C.

Elemental analysis for $C_{42}H_{42}Cl_7N_8O_{18}P$
Found, %: C 41.12, H 3.28, Cl 20.36, N 9.00, P 2.30.
Calculated, %: C 41,14, H 3.45, Cl 20.24, N 9.14, P 2.52.
IR spectrum for N=P : 1360 $cm^{-1}$.

The compound had a virulicidal activity. The composition based thereon was tested to influenza A viruses according to a manner similar to that described in Example 21. The test results are given in Table 4.

Example 29

To a solution containing 0.06 mol of aniline in 50 ml of benzene 0.01 mol of trichlorophosphazo-1-para-nitrophenyl-1,3-propandiol in 30 ml of benzene was gradually added under stirring. The reaction mixture was allowed to stay for 24 hours at 20° C. The precipitated hydrochloric aniline was filtered off. Bensene was evaporated in vacuum. The residue containing the product as a viscous substance was purified by reprecipitating it from a benzene solution using a petroleum ether in the presence of an activated charcoal. The obtained end product namely trianilidophosphazo-1-para-nitrophenyl-1,3-propanediol of the formula:

para-$NO_2C_6H_4CHOHCH/CH_2OH/N=P/NHO_6H_5/_3$

was a yellow viscous liquid, which was purified by repricipitation of it from a benzene solution with a petroleum ether in the presence of an activated charcoal, soluble in water, alcohols, acetone, benzene, toluene. The yield of the end product was 88% by mass.

Elemental analysis for $C_{27}H_{28}N_5O_4P$
Found. %: C 62.96, H 5.19, N 13.36, P 5.92.
Calculated, %: C 62.66, H 5.45, N 13.50, P 6.00.
IR spectrum for N=P : 1345 $cm^{-1}$.

The obtained compound had an ovocidal activity. Based on the compound there was prepared an ovocidal composition which was a 2% aqueous solution based thereon. The activity of an ovocidal composition of the invention was tested for disinfection of environmental objects / equipment, tableware, hands/. For this purpouse environmental objects were treated with a 2% aqueous solution of the compound according to the invention. For the control there were used objects which were treated with water without amploying the composition. The ovocidal efficiency of the composition was determined according to a number of helminthic eggs presenting in the washings from the environmental objects before and after treating them with the composition. For a test object there were used pig ascarid eggs which were the most stable among helminths in the environment. A suspension droplet containing at least 1000 ascaridal eggs was placed onto watch glasses followed by adding 1 droplet of a 2% solution of the composition according to the invention thereto, and thereafter they were put into a humid compartment of the thermostat at a temperature in the range of 24 to 26 °C. 24 hours for 3 days the watch glasses with ascaridal eggs and the composition of the invention were taken away from the autoclave and a 2-3-fold washed with a distilled water. Thereafter, the washed ascaridal eggs were cultured for 30 days at 24-26 °C. To determine a number of killed and vital eggs 20 eggs were observed by using a microscope in every 3-5 days. The death was determined by lacking the egg development for 30 days to the stage of a mobile larva as well as by presence of destructive morphological alterations: an embryo wrinkling and deformation, the breakage of envelopes. The test results are given in Table 5 in comparison with a known composition (carbathions) and the control.

Example 30

The process was carried out in a manner similar to that described in Example 29 except that 0.02 mol of aniline and 0.01 mol of trichlorophosphazo-1-para-nitrophenyl-1,3-dichloropane were used as the starting compounds. The obtained end product or monoanilidodichlorophosphaszo-1-para-nitrophenyl-1,3-dichloropropane of the formula:

para-$NO_2C_6H_4CHClCH/CH_2Cl/N=P/N\ HC_6H_5/Cl_2$

was a yellow viscous liquid, soluble in water, alcohols, acetone, benzene, toluene. The yield of the end product was 85% by mass.

Elemental analysis for $C_{15}H_{14}Cl_4N_3O_2P$

Found, %: C 40.61, H 3.04, Cl 32.00, N 9.46, P 7.18.

Calculated, %: C 40.85, H 3.20, Cl 32.15, N 9.52, P 7.02.

IR spectrum for N=P : 1340 $cm^{-1}$.

The compound had an ovocidal activity. The composition based thereon was tested in a manner similar to that described in Example 29. The test results are given in Table 5.

Table 4

Virulicidal activity of the composition according to the invention over a known composition sodium N-chlorob enzenesulfamide

| Nos. | Compositions | Influenza A viruses: Concentration of the composition, % by mass | Virulicidal activity (%) under exposure: 30 min | 60 min |
|---|---|---|---|---|
| 1 | 2 | 3 | 4 | 5 |
| 1. | The composition of the invention according to Example 21 | 1.0 | 87.5 | 100.0 |
| | | 2.0 | not tested | |
| 2. | The composition of the invention according to Example 22 | 1.0 | 12.5 | 50.0 |
| | | 2.0 | 50.0 | 75.0 |

Table 4 (continued)

| 1 | 2 | 3 | 4 | 5 |
|---|---|---|---|---|
| 3. | The composition of the invention according to Example 23 | 1.0 | 75.0 | 98.0 |
| | | 2.0 | 93.8 | 100.0 |
| 4. | The composition of the invention according to Example 24 | 1.0 | 50.0 | 75.0 |
| | | 2.0 | 93.8 | 100.0 |
| 5. | The composition of the invention according to Example 25 | 1.0 | 75.0 | 93.8 |
| | | 2.0 | 93.8 | 100.0 |
| 6. | The composition of the invention according to Example 26 | 1.0 | 50.0 | 75.0 |
| | | 2.0 | 98.4 | 100.0 |
| 7. | A known composition | 1.0 | 12.5 | 25.0 |
| | | 2.0 | 25.0 | 50.0 |
| 8. | The composition of the invention according to Example 27 | 1.0 | 100.0 | |
| | | 2.0 | 100.0 | |
| 9. | The composition of the invention according to Example 28 | 1.0 | 100.0 | |
| | | 2.0 | 100.0 | |
| 10. | A known composition | 1.0 | 22.0 | |
| | | 2.0 | 25.1 | |

Table 4 (continued)

| Nos | Compositions | Hepatitis B virus | | |
|---|---|---|---|---|
| | | Concentration of the composition, % by mass | Virulicidal activity (%) under exposure | |
| | | | 30 min | 60 min |
| 1. | The composition of the invention according to Example 21 | 1.0 | 50.0 | 87.5 |
| | | 2,0 | 100,0 | 100,0 |
| 2. | The composition of the invention according to Example 22 | 1.0 | 12.5 | 50.0 |
| | | 2,0 | 25,0 | 75,0 |
| 3. | The composition of the invention according to Example 23 | 1.0 | 100.0 | 100.0 |
| | | 2,0 | 100,0 | 100,0 |
| 4. | The composition of the invention according to Example 24 | 1.0 | 75.0 | 100.0 |
| | | 2,0 | 100,0 | 100,0 |
| 5. | The composition of the invention according to Example 25 | 1.0 | 100.0 | 100.0 |
| | | 2,0 | 100,0 | 100,0 |
| 6. | The composition of the invention according to Example 26 | 1.0 | 50.0 | 75.0 |
| | | 2,0 | 100,0 | 100,0 |
| 7. | A known composition | 1.0 | 9.0 | 25.0 |
| | | 2.0 | 12.5 | 50.0 |
| 8. | The composition of the invention according to Example 27 | | - | - |
| 9. | The composition of the invention according to Example 28 | | - | - |
| 10. | A known composition | | | |

Table (continued)

| Nos. | Compositions | Meningopneumonia virus: Concentration of the composition: % by mass | Virulicidal activity (%) under exposure: 30 min | 60 min |
|---|---|---|---|---|
| 1. | The composition of the invention according to Example 21 | | - | - |
| 2. | The composition of the invention according to Example 22 | 0.1 | 10.0 | 50.0 |
| | | 0,2 | 25,0 | 75,0 |
| 3. | The composition of the invention according to Example 23 | 0.1 | 99.0 | 100.0 |
| | | 0,2 | 100,0 | 100,0 |
| 4. | The composition of the invention according to Example 24 | 0.1 | 75.0 | 100.0 |
| | | 0,2 | 99,0 | 100,0 |
| 5. | The composition of the invention according to Example 25 | 0.1 | 50.0 | 75.0 |
| | | 0,2 | 90,0 | 100,0 |
| 6. | The composition of the invention according to Example 26 | 0.1 | 25.0 | 50.0 |
| | | 0,2 | 50,0 | 99,0 |
| 7. | A known composition | 0,1 | 10,0 | 25,0 |
| | | 0.2 | 25.0 | 50.0 |
| 8. | The composition of the invention according to Example 27 | | - | - |
| 9. | The composition of the invention according to Example 28 | | - | - |
| 10. | A known composition | | | |

Example 31

Similarly to that described in Example 1 tri-n-propoxyphosphazo-1-para-nitrophenyl-1-chloro-3-propanol was obtained. The compound had an ovocidal activity. The composition based thereon tested in a manner similar to that described in Example 29. The test data are given in Table 5.

Example 32

According to a manner similar to that described in Example 27, there was obtained tris-[D(-)-threo-3-paranitrophenyl-2-dichloroacetamido-2-hydroxypropoxy]phosphazo-L( + )-threo-1-para-nitrophenyl-1,3-dihydroxyisopropyl. The compound had an ovocidal activity. The composition based thereon was tested in a manner similar to that described in Example 29. The test data are given in Table 5,

Example 33

Similarly to that described in Example 28 tris[D(-)-threo-3-para-nitrophenyl-2-dichloroacetamido-2-hydroxypropoxy] phosphazo-L( + )-threo-1-para-nitrophenyl-1-hydroxy-3-chloroisopropyl was obtained. The compound had an ovocidal activity. The composition based thereon was tested in a manner similar to that described in Example 29. The test results are given in Table 5.

Examples 34-35

According to a manner similar to that described in Examples 27-28 there were obtained tris[D(-)-threo-3-paranitrophenyl-2-dichloroacetamido-2-hydroxypropoxy] phosphazo-L( + )-threo-1-para-nitrophenyl-1,3-dihydroxyisopropyl and tris [D(-)-threo-3-para-nitrophenyl-2-dichloroacetamido-2-hydroxypropoxy] phosphazo-L( + )-threo-1-para-nitrophenyl-1-hydroxy-3-chloroisopropyl. The compounds obtained had insecticidal activity. The tests of the insecticidal compositions based thereon were carried out in a manner similar to that described in Example 1. The test results are given in Table 6.

Table 5

Ovocidal activity of the composition according to the invention over a known composition (carbathione) and the control

| Nos | Compositions | Concentration of the composition, % by mass | Death of ascaridal eggs, % | |
|---|---|---|---|---|
| | | | after 24 hours | after 48 hours |
| 1. | The composition of the invention according to Example 29 | 2.0 | 97.6 | 100.0 |
| 2. | The composition of the invention according to Example 30 | -"- | 100.0 | 100.0 |
| 3. | The composition of the invention according to Example 31 | -"- | 100.0 | 100.0 |
| 4. | The composition of the invention according to Example 32 | -"- | 100.0 | 100.0 |
| 5. | The composition of the invention according to Example 33 | -"- | 100.0 | 100.0 |
| 6. | A known composition | -"- | 60.0 | 80.0 |
| 7. | Control | - | 0.0 | 0.0 |

Table 6

Insecticidal activity of the composition according to the invention over a known composition based on O.O-dimethyl-/1-hydroxy-2,2,2-trichloroethyl/phosphonate

| Nos. | Compositions | Concentration, % by mass | Death of flies, % | |
|---|---|---|---|---|
| | | | after 48 hours | after 72 hours |
| 1. | The insecticidal composition of the invention according to Example 34 | 2.0 | 34.0 | 62.0 |
| 2. | The insecticidal composition of the invention according to Example 35 | 20.0 | 36.0 | 89.0 |
| 3. | A known composition | 20.0 | 81.0 | 84.0 |

Example 36

To a solution containing 0.02 mol of phenol and 0.02 mol of triethylamine in 30 ml of benzene 0.01 mol of trichlorophosphazo-1-para-nitrophenyl-1,3-dihydroxyisopropyl in 20 ml of benzene was gradually added under stirring. The teaction mixture was allowed to stay at 20° C for 24 hours. The precipitated hydrochloric triethylamine was sucked off. Benzene was evaporated in vacuum. In the residue there was obtained the product as a viscous liquid which was purified by reprecipitating it from benzene with petroleum ether in the presence of an activated charcoal. As a result, there was obtained diphenoxychlorophosphazo-1-nitrophenyl-1,3-dihydroxypropyl of the following formula;

para-$NO_2C_6H_4CHOHCH/CH_2OH/N=P/OC_6H_5/_2Cl$.

The yield of the end product was 87% by mass.

Elemental analysis for $C_{21}H_{20}ClN_2O_6P$

Found, %: C 54.31, H 4.43, Cl 7.58, N 5.87, P 6.52.

Calculated, %: C 54.49, H 4.35, Cl 7.66, N 6.05, P 6.69.

IR spectrum for N=P : 1330 $cm^{-1}$.

The obtained compound had a bactericidal and a virulicidal activity. The bactericidal composition based thereon was tested according to a manner similar to that described in Example 9. The test results are given

in Table 7. The virulicidal composition based thereon was tested according to a manner similar to that described in Example 21. The test data are given in Table 8.

Example 37

Similarly to that described in Example 36, there was obtained di-para-chlorophenoxychlorophosphazo-1-para-nitrophenyl-1,3-dihydroxyisopropyl of the following formula:
para-$NO_2C_6H_4CHOHCH/CH_2OH/N=P/OC_6H_4Cl$-para/$_2Cl$
The yield of the end product was 84% by mass.

Elemental analysis for $C_{21}H_{18}Cl_3N_2O_6P$
Found, %: C 46.95, H 3.34, Cl 19.68, N 5.32, P 5.51.
Calculated, %: C 47.43, H 3.41, Cl 20.0, N 5.26, P 5.82.
IR spectrum for N=P : 1340 $cm^{-1}$.

The obtained compound had a bactericidal and virulicidal activity.
The test of the bactericidal composition based thereon was conducted according to a manner similar to that of Example 9. The test results are given in Table 7. The virulicidal composition based thereon was tested according to a manner similar to that of Example 21. The test results are given in Table 8.

Example 38

Similarly to that described in Example 36, there was obtained phenoxydichlorophosphazo-1-para-nitrophenyl-1,3-dihydroxyisopropyl of the following formula:
para-$NO_2C_6CHOHCH/CH_2/N=P/OC_6H_5/Cl$
The yield of the end product was 86% by mass.

Elemental analysis for $C_{15}H_{15}Cl_2N_2O_5P$
Found, %: C 43.67, H 3.89, Cl 17.22, N 6.74, P 7.30.

Calculated, %: C 44.47, H 3.73, Cl 17.50, N 6.91, P 7.64.
IR spectrum for N=P : 1335 $cm^{-1}$.

The obtained compound had a bactericidal and a virulicidal activity. The tests of a bactericidal and a virucilidal composition based thereon were carried out in a manner similar to that described in Example 9 and 21, respectively. The test results are given in Tables 7-8.

Example 39

According to a manner similar to that described in Example 36 para-chlorophenoxydichlorophosphazo-1-para-nitrophenyl-1,3-dihydroxyisopropyl of the following formula:
para-$NO_2C_6H_4CHOHCH/CH_2OH/N=P/OC_6H_4Cl$-para/$Cl_2$ was obtained.
The yield of the end product was 98% by mass.

Elemental analysis for $C_{15}H_{14}Cl_3N_2O_5P$
Found, %: C 40.89, H 3.14, Cl 23.87, N 6.22, P 6.79.
Calculated, %: C 40.98, H 3.21, Cl 24.19, N 6.37, P 7.04.
IR spectrum for N=P : 1340 $cm^{-1}$.

The obtained compound had a bactericidal and a virulicidal activity. The bactericidal and virulicidal compositions based thereon were tested according to a manner similar to that described in Examples 9 and 21, respectively. The test data are given in Tables 7-8.

Example 40

Similarly to that described in Example 36 there was obtained tri-para-methylphenoxy-1-para-nitrophenyl-1,3-dihydroxyisopropyl of the following formula:
para-$NO_2C_6H_4CHOHCH/CH_2OH/N = P/OC_6H_4CH_3$-para/$_3$
The yield of the end product was 86% by mass.

Elemental analysis for $C_{30}H_{31}N_2O_7P$
Found, %: C 63.72, H 5.61, B 5.04, P 5.38.
Calculated, %: C 64.05, H 5.55, N 4.97, P 5.50.
IR spectrum for N=P : 1335 $cm^{-1}$.

The obtained compound had a bactericidal and a virulicidal activity. The test of a bactericidal and a virulicidal composition based thereon was carried out according to a a manner similar to that described in Examples 9 and 21, respectively. The test results are shown in Tables 7-8.

Example 41

According to a manner similar to that described in Example 36, there was obtained tri-para-chlorophenoxyphosphazo-1-para-nitrophenyl-1,3-dihydroxyisopropyl of the following formula:
para-$NO_2C_6H_4CHOHCH/CH_2OH/N = P/OC_6H_4Cl$-para/$_3$.
The yield of the end product was 80% by mass.

Elemental analysis for $C_{27}H_{22}Cl_3N_2O_7P$
Found, %: C 51.76, H 3.41, Cl 17.10, N 4.38, P 4.84.
Calculated, %: C 51.98, H 3.55, Cl 17.84, N 4.49, P 4.96.
IR spectrum for N=P : 1340 $cm^{-1}$.

The compound obtained had a virulicidal and a bactericidal activity. The tests of a bactericidal and a virulicidal composition based thereon were carried out according to a manner similar to that described in Examples 9 and 21, respectively. The test results area shown in Tables 7-8.

Table 7

Bactericidal activity of the composition according to the invention over a known composition based on sodium N-chlorobenzenesulphamide

| Nos. | Compositions | Concentration, % by mass | Death of microorganisms, % | |
|---|---|---|---|---|
| | | | E.coli | S.aureus |
| 1. | The bactericidal composition of the invention according to Example 36 | 0.2 | 100.0 | 90.0 |
| 2. | -"- according to Example 37 | 0.2 | 100.0 | 100.0 |
| 3. | -"- according to Example 38 | 0.2 | 100.0 | 95.0 |
| 4. | -"- according to Example 39 | 0.2 | 100.0 | 100.0 |
| 5. | -"- according to Example 40 | 0.2 | 90.0 | 70.0 |
| 6. | -"- according to Example 41 | 0,2 | 100,0 | 90,0 |
| 7. | A known composition | 0.2 | 100.0 | 90.0 |

Virulicidal activity of the composition according to the invention over a known composition based sodium N-chlorobenzenesulphamide

| Nos | Compositions | Concentration, % by mass | Virulicidal activity (%) to poliomyelitis virus: under exposure of 30 min | under exposure of 60 min |
|---|---|---|---|---|
| 1. | The virulicidal composition of the invention according to Example 36 | 1.0 | 50.0 | 80.0 |
| | | 2.0 | 90.0 | 90.0 |
| 2. | -"- according to Example 37 | 1.0 | 60.0 | 90.0 |
| | | 2.0 | 90.0 | 100.0 |
| 3. | -"- according to Example 38 | 1.0 | 60.0 | 90.0 |
| | | 2.0 | 90.0 | 95.0 |
| 4. | -"- according to Example 39 | 1.0 | 70.0 | 90.0 |
| | | 2.0 | 95.0 | 100.0 |
| 5. | -"- according to Example 40 | 1.0 | 50.0 | 70.0 |
| | | 2.0 | 80.0 | 90.0 |
| 6. | -"- according to Example 41 | 1.0 | 50.0 | 80.0 |
| | | 2.0 | 90.0 | 100.0 |
| 7. | A known composition | 1.0 | 9.0 | 25.0 |
| | | 2.0 | 12.5 | 50.0 |

Industrial Applicability

The novel compounds of the invention namely trichlorophosphazohydrocarbon derivatives, and insecticidal, bactericidal, fungicidal, virulicidal and ovocidal compositions based thereon find application in medicine, veterinary and agriculture for controlling against synanthropic insects in living and industrial premises, farms as well as for disinfection of implements, dishware, medicinal instruments, clothes, any garbage, waste waters, and rooms of isolation hospitals, out-patient's clinics, veterinary surgeries, living rooms, farms and the like from bacteria, fungi, viruses, microorganisms and helminths.

## Claims

1. Trichlorophosphazohydrocarbon derivatives of the general formula:
para-$NO_2C_6H_4CHXCH/CH_2X'/N = PRR^1R^2$, wherein X and X' each represent hydroxyl or chlorine, $P = R^1 = R^2$ are $OCH_3$, $OC_2H_5$, $OC_3H_7$-H, $OC_3H_7$-iso, $OC_4H_9$-H, $OCH/C_6H_5/CH_2Cl$.

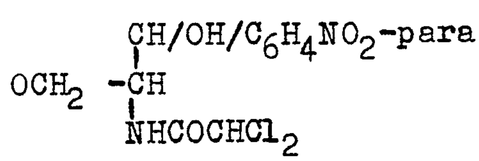

$OC_6H_4CH_3$-para, $OC_6H_4Cl$-para, wherein when R is $OC_6H_5$, $OC_6H_4Cl$-para, then $R = R^2$ are Cl or $OC_6H_5$, and when R is $NHC_6H_5$, $R^1 = R^2$ each represent Cl or $NHC_6H_5$: and when $R = R^1$ each represent $OC_6H_5$, $OC_6H_4Cl$-para, $R^2$ is Cl.

**2.** An insecticidal composition comprising an active ingredient and a solvent, characterized in that as an active ingredient it contains trichlorophosphazohydrocarbon derivatives of the general formula;
para-$NO_2C_6H_4CHXCH/CH_2X'/N = PRR^1R^2$, wherein
X and X' each represent hydroxyl or chlorine,
$R = R^1 = R^2$ each represent $OCH_3$, $OC_2H_5$, $OC_3H_7$-H, $OC_3H_7$-iso, $OC_4H_9$-H, $OCH/C_6H_5/CH_2Cl$,

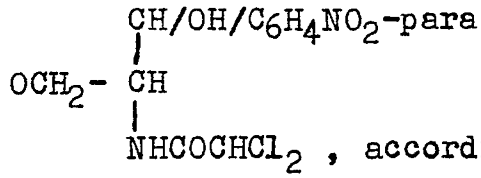
, according to Claim 1.

**3.** An insecticidal composition according to Claim 1, characterized in that it contains an active ingredient in the amount of 2% by mass.

**4.** An insecticidal composition according to Claim 2, characterized in that as a solvent it contains a 5% aqueous glucose solution.

**5.** A bactericidal composition comprising an active ingredient and a solution characterized in that as an active ingredient it contains trichlorophosphazohydrocarbon of the general formula;
para-$NO_2C_6H_4CHXCH/CH_2X'/N = PRR^1R^2$, wherein
X and X' each represent hydroxyl or chlorine, $R = R^1 = R^2$ are $OCH_3$, $OC_2H_5$, $OC_6H_4CH_3$-para, $OC_6H_4Cl$-para; provided that R is $OC_6H_5$ and $OC_6H_4Cl$-para, $R^1 = R^2$ each represent Cl or $O_2C_6H_5$; and when $R = R^1$ each represent $OC_6H_5$, $OC_6H_4Cl$-para, $R^2$ is Cl, according to Claim 1.

**6.** A bactericidal composition according to Claim 5, characterized in that it contains an active ingredient in the amount of 0.2 to 0.5% by mass.

**7.** A bactericidal composition according to Claim 5, characterized in that it contains water as a solvent.

**8.** A fungicidal composition comprising an active ingredient and a solvent characterized in that as an active ingredient it contains trichlorophosphazohydrocarbon derivatives of the general formula:
para-$NO_2C_6H_4CHXCH/CH_2X'/N = PRR^1R^2$, wherein
X and X' each represent hydroxyl or chlorine, $R = R^1 = R^2$ are $OCH_3$, $OC_2H_5$, according to Claim 1.

**9.** A fungicidal composition according to Claim 8, characterized in that it contains an active ingredient in the amount of 0.01% by mass.

**10.** A fungicidal composition according to Claim 8, characterized in that it contains water as a solvent

**11.** A virulicidal composition comprising an active ingredient and a solvent characterized in that as an active ingredient it contains trichlorophosphazocarbon derivatives of the general formula:
para-$NO_2C_6H_4CHXCH/CH_2X'/N = PRR^1R^2$, wherein
X and X' each represent hydroxyl or chlorine,
$R = R^1 = R^2$ are $OC_2H_5$, $OC_3H_7$-H, $OC_3H_7$-iso, $OC_4H_9$-H, $OCH/C_6H_5/CH_2Cl$.

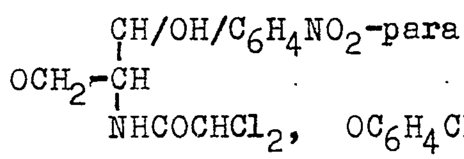
, $OC_6H_4CH_3$-para,

$OC_6H_4Cl$-para; provided that R is $OC_6H_5$, $OC_6H_4Cl$-para, $R^1 = R^2$ each represent Cl or $OC_6H_5$, and when $R = R^1$ are $OC_6H_5$, $OC_6H_4Cl$-para, $R^2$ is chlorine, according to Claim 1.

**12.** A virulicidal composition according to Claim 11, characterized in that it contains an active ingredient in the amount of 0.1 ` to 27co by mass.

**13.** A Arulicidelcomposition according to ClaimlI, characteAzedin that as,asolvent it contains a 0.85% sodium chloride solution.

**14.** An ovocidal composition comprising an active ingredient and a solvent characterized in that as an active ingredient it contains trichlorophosphazohydrocarbon derivatives of the general formula: para-$NO_2C_6H_4CHXCH/CH_2X'/N = PRR^1R^2$, wherein X and X' each represent hydroxyl or chlorine, $P = R^1 = R^2$ are $OC_6H_5$,

$CH/OH/C_6H_4NO_2$-para
|
$OCH_2-$ CH
|
$NHCOCHCl_2$

and when R is $HC_6H_5$, $R^1 = R^2$ are Cl or $NHC_6H_5$, according to Claim 1.

**15.** An ovocidal composition according to Claim 14, characterized in that it contains an active ingredient in the amount of 2% by mass.

**16.** An ovocidal composition according to Claim 14, characterized in that as a solvent it contains water.

# INTERNATIONAL SEARCH REPORT

International Application No PCT/SU 89/00026

**I. CLASSIFICATION OF SUBJECT MATTER** (if several classification symbols apply, indicate all) [6]

According to International Patent Classification (IPC) or to both National Classification and IPC

Int. Cl.[5] C07F 9/09, 9/11, 9/12, 9/14, 9/24, C07F 9/26, A61K 31/675, A01N 57/30

**II. FIELDS SEARCHED**

Minimum Documentation Searched [7]

| Classification System | Classification Symbols |
|---|---|
| Int. Cl.[4] | C07F 9/09, 9/11, 9/12, 9/14, 9/24, 9/26, A01N 57/28, 57/30, A61K 31/675 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | Fiziologicheski aktivnye veschestva, sbornik, vyp. 4, 1972, Naukova dumka, (Kiev), E.A. Shomova et al "Fungitsidnaya aktivnost triamidofosfazogaloidokarbatsilov", see pages 15-17 | 1 |
| A | SU, A1, 666810 (Institut fizicheskoi khimii), 25 February 1980 (25.02.80) | 2-16 |
| A | US, A, 4341772 (E.I. DU PONT de NEMOURS AND COMPANY), 27 July 1982 (27.07.82) | 2-16 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 19 September 1989 (19.09.89) | 12 October 1989 (12.10.89) |
| **International Searching Authority** | **Signature of Authorized Officer** |
| ISA/SU | |